## Europäisches Patentamt

## European Patent Office

## Office européen des brevets

(19)

(11) Veröffentlichungsnummer: **0 175 117**
**B1**

(12)

# EUROPÄISCHE PATENTSCHRIFT

(45) Veröffentlichungstag der Patentschrift:
29.03.89

(51) Int. Cl.⁴: **C 07 C 118/00**

(21) Anmeldenummer: **85109810.3**

(22) Anmeldetag: **05.08.85**

(54) **Verfahren zur Gewinnung von reinen Diisocyanaten.**

(30) Priorität: **16.08.84 DE 3430022**

(43) Veröffentlichungstag der Anmeldung:
**26.03.86 Patentblatt 86/13**

(45) Bekanntmachung des Hinweises auf die Patenterteilung:
**29.03.89 Patentblatt 89/13**

(84) Benannte Vertragsstaaten:
**BE DE FR GB IT**

(56) Entgegenhaltungen:
**DE-A-1 543 258**
**DE-B-1 243 178**
**FR-A-2 279 723**

(73) Patentinhaber: **BAYER AG, Konzernverwaltung RP Patentabteilung, D-5090 Leverkusen 1 Bayerwerk (DE)**

(72) Erfinder: **Molnar, Attila, Dr., Osterende 9, D-2211 Brockdorf (DE)**
Erfinder: **Zarnack, Uwe, Dr., Ulitzhörn 2, D-2212 Brunsbüttel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft ein verbessertes Verfahren zur Gewinnung von reinen Diisocyanaten ausgewählt aus der Gruppe bestehend aus Diisocyanatotoluol, Diisocyanatohexan und 1-Isocyanato-3,3,5-trimethyl-5-isocyanatomethyl-cyclohexan (Isophorondiisocyanat) aus dem bei der Herstellung der Diisocyanate durch Phosgenierung der ihnen zugrundeliegenden Diamine anfallenden Rohlösungen durch Extraktion der aus den Rohlösungen hergestellten Konzentrate.

Aus der US-PS-4 372 891 ist bereits bekannt, daß Destillationsrückstände von Diisocyanatotoluol (TDI), die noch monomeres TDI enthalten, aber durch Destillation nicht weiter eingeengt werden können, mit einem Lösungsmittelgemisch aus aliphatischen und aromatischen oder aliphatischen und chlorierten Kohlenwasserstoffen extrahiert werden können, um monomeres TDI zurückzugewinnen. Hierbei werden polymere Rückstandsprodukte in fester Form ausgeschieden. Bei diesem, auf die Aufarbeitung von Diisocyanatotoluol-Destillationsrückständen beschränkten Verfahren können nur TDI-Destillationsrückstände eingesetzt werden, die einen hohen Polymerisatanteil aufweisen. Außerdem können bei diesem Verfahren als Extraktionsmittel nur Gemische unterschiedlicher Lösungsmittel zum Einsatz kommen. Nachteilig ist außerdem die Ausscheidung der polymeren Rückstandsverbindungen in fester Form, da eine kontinuierliche Abtrennung von Feststoffen, verglichen mit einer kontinuierlichen Abtrennung von Flüssigkeiten nur mit einem erhöhten technischen Aufwand realisierbar ist.

Die US-PS-3 211 631 lehrt, daß TDI-Destillationsrückstände mit Extraktionsmitteln wie beispielsweise Pentan oder Hexan extrahiert werden können. Die hierbei zum Einsatz gelangenden TDI-Destillationsrückstände müssen jedoch vor der Extraktion von dem bei ihrer Herstellung verwendeten Lösungsmittel befreit werden. Dieses Verfahren weist zwei Nachteile auf. Einerseits muß ein erheblicher Destillationsaufwand getrieben werden, um das bei der Phosgenierung verwendete Lösungsmittel vollständig vom Rohisocyanat zu entfernen und andererseits ist es unumgänglich, die Extraktion unter Druck auszuführen, wenn erhöhte Temperaturen angewandt werden sollen, die oberhalb der Siedetemperaturen der bevorzugten Extraktionsmittel liegen. Insbesondere bei mehrstufigen Extraktionen sind in den höheren Extraktionsstufen jedoch Temperaturen zwischen 60°C und 130°C zweckmäßig, um die Ausbeute an Extraktionsgut zu erhöhen und gleichzeitig die Viskosität des anfallenden, flüssigen Rückstands zu senken.

In der US-PS-3 144 474 wird die Reinigung roher Phosgenierlösungen mit Rohisocyanatgehalten von 30 bis 60 % in chlorierten organischen Lösungsmitteln durch Ausfällung von Verunreinigungen mit Petroleum-Fraktionen beschrieben. Die Verunreinigungen können bis zu 25 % des Isocyanatgewichtes der Rohlösung umfassen und werden durch Filtration abgetrennt. Weitere Aufkonzentrierungen der rohen Isocyanate werden als nicht handhabbar beschrieben. Nachteilhaft ist auch bei diesem Verfahren die Feststoffabtrennung aus der Rohlösung.

Die US-PS-3 987 075 beschreibt die Extraktion von Destillationsrückständen, wie sie bei der destillativen Aufarbeitung von Lösungen erhalten werden, die bei der Phosgenierung von organischen Aminen in inerten Lösungsmitteln anfallen. Als Extraktionsmittel werden alkylsubstituierte Benzole eingesetzt, die Extraktion erfolgt bei einer Temperatur im Temperaturbereich von 130 bis 280°C, wobei das zu extrahierende, rückstandshaltige Isocyanatgemisch vor der Phasentrennung 0,5 - 25 h mit dem spezifischen Extraktionsmittel behandelt wird. Hierbei anfallende Feststoffe müssen durch Filtration entfernt werden. Ein weiterer Nachteil dieses Verfahrens besteht darin, daß die Extraktion bei einer sehr hohen Temperatur durchgeführt wird, bei welcher bereits thermische Zersetzungen und Umsetzungen mit Rückstandskomponenten der zu extrahierenden Isocyanate stattfinden, so daß die Menge der extrahierbaren Isocyanate und somit die Gesamtausbeute reduziert wird.

Die DE-OS-1 543 258 beschreibt die Extraktion roher Polyisocyanat-Phosgeniergemische mit einem Isocyanatgehalt von vorzugsweise 5 - 15 Vol.-% mit einem Lösungsmittelgemisch aus zwei unterschiedlichen Lösungsmitteln, die am Extraktionssystem mit mehr als 75 Vol-% beteiligt sein müssen. Dabei sollen mindestens 5 % des Extraktionssystems aus einem Lösungsmittel bestehen, in dem die Phosgenierungsprodukte löslich sind. Der Rest des Lösungsmittelgemisches soll ein Nichtlöser für die hochmolekularen Homologen und Nebenprodukte der Phosgenierung sein. Ziel des Verfahrens ist die Gewinnung gereinigter, praktisch nicht flüchtiger Polyisocyanate. Nachteil des Verfahrens ist die Verwendung zweier verschiedener Lösungsmittel in einem definierten Mengenverhältnis der Komponenten, um eine Phasentrennung zu realisieren.

Gemäß DE-OS-2 532 722 werden Polyisocyanatgemische der Diphenylmethanreihe, die vorher bereits von dem bei ihrer Herstellung verwendeten Lösungsmittel befreit worden sind, und die mindestens 80 Gew.-% 4,4'-Diisocyanatodiphenylmethan enthalten, bei erhöhten Temperaturen in aliphatischen Kohlenwasserstoffen gelöst, worauf sich eine Abkühlung der Lösung unter Bildung eines zweiphasigen Systems anschließt. Das gereinigte 4,4'-Diisocyanatodiphenylmethan wird aus der so erhaltenen oberen Schicht gewonnen.

Es gehört somit zum Stand der Technik, die Abtrennung von monomeren organischen Isocyanaten aus Polyisocyanatlösungen oder aus Isocyanatprodukten, in denen monomere organische Isocyanate gelöst sind, so durchzuführen, daß die abzutrennenden monomeren Isocyanate im Vakuum bei hoher Temperatur möglichst vollständig abdestilliert werden. Die dabei anfallenden Destillationsrückstände können dann zur weiteren Gewinnung von Isocyanaten einer Extraktion zugeführt werden.

Es war die der Erfindung zugrundeliegende Aufgabe, ein neues Verfahren zur Herstellung von reinen Diisocyanaten aus den bei ihrer Herstellung durch Phosgenierung der ihnen zugrundeliegenden Diamine anfallenden Lösungen zur Verfügung zu stellen, welche nicht mit den genannten Nachteilen der Verfahren des Standes der Technik behaftet ist. Dies bedeutet insbesondere, daß das Verfahren bei Temperaturen von maximal 130°C durchführbar sein sollte, gleichzeitig mit einem vergleichsweise geringen destillativen Aufwand verbunden ist und außerdem die Bildung von festen Nebenprodukten ausschließt, so daß eine kontinuierliche Arbeitsweise ohne technische Schwierigkeiten ermöglicht wird.

Diese Aufgabe konnte durch das nachstehend näher beschriebene erfindungsgemäße Verfahren gelöst werden.

Gegenstand der Erfindung ist ein Verfahren zur Gewinnung von reinen Diisocyanaten ausgewählt aus der Gruppe bestehend aus (i) 2,4-Diisocyanatotoluol und dessen Gemischen mit bis zu 35 Gew.-%, bezogen auf Gesamtgemisch, an 2,6-Diisocyanatotoluol, (ii) 1,6-Diisocyanatohexan und (iii) 1-Isocyanato-3,3,5-trimethyl-5-isocyanatomethyl-cyclohexan aus konzentrierten Lösungen der Phosgenierprodukte der den Diisocyanaten zugrundeliegenden Diamine in technischem Chlorbenzol und/oder technischem ortho-Dichlorbenzol, dadurch gekennzeichnet, daß man die bei der Phosgenierung der den Diisocyanaten zugrundeliegenden Diamine in den genannten Lösungsmitteln anfallenden, einen Lösungsmittelgehalt von 40 bis 98 Gew.-% aufweisenden Lösungen der Phosgenierungsprodukte destillativ von der Hauptmenge des Lösungsmittels und gegebenenfalls einer Teilmenge des gebildeten monomeren Diisocyanats bis zu einem Restgehalt des Lösungsmittels von 2 bis 15 Gew.-% befreit, die so erhaltenen Konzentrate bei Temperaturen zwischen -20°C und 130°C mit bei Raumtemperatur flüssigen aliphatischen Kohlenwasserstoffen und/oder mit bei Raumtemperatur flüssigen aliphatischen Ethern als Extraktionsmittel unter Einhaltung eines Gewichtsverhältnisses von Extraktionsmittel zu Konzentrat von 0,5 : 1 bis 30 : 1 innig unter Bildung eines flüssigen, zweiphasigen Gemischs vermischt, die sich bildenden Flüssigphasen nach Phasenabscheidung voneinander trennt, das reine Diisocyanat aus der spezifisch leichteren Flüssigphase durch destillative Entfernung des in ihr enthaltenen Lösungs- und Extraktionsmittels gewinnt und gegebenenfalls die abgetrennte, spezifisch schwere Phase einer erneuten Extraktion zuführt.

Beim erfindungsgemäßen Verfahren werden Lösungen eingesetzt, wie sie bei der Phosgenierung der in reiner Form zu gewinnenden Diisocyanate entsprechenden Diamine in Chlorbenzol oder ortho-Dichlorbenzol nach den bekannten Phosgenierungsverfahren des Standes der Technik (ein- oder zweistufige Phosgenierung der Diamine, ihrer Hydrochloride oder ihrer Kohlendioxid-Addukte) anfallen. Es handelt sich bei den Ausgangsmaterialien somit um technische Lösungen der Phosgenierungsprodukte von (i) 2,4-Diaminotoluol oder dessen Gemischen mit bis zu 35 Gew.-%, bezogen auf Gesamtgemisch an 2,6-Diaminotoluol, (ii) 1,6-Diaminohexan oder (iii) 1-Amino-3,3,5-trimethyl-5-aminomethyl-cyclohexan bzw. ihrer Hydrochloride oder Kohlendioxid-Addukte. Diese Rohlösungen weisen einen Gehalt an Lösungsmittel (Chlorbenzol und/oder ortho-Dichlorbenzol von 40 bis 98 Gew.-%) auf. Außerdem enthalten diese Lösungen neben dem in reiner Form zu gewinnenden Diisocyanat unterschiedliche Mengen an Nebenprodukten, insbesondere oligomere oder polymere Bestandteile, die während der Phosgenierungsreaktion gebildet werden, und die von den Diisocyanaten abgetrennt werden müssen. Der Anteil dieser zu entfernenden Nebenprodukte kann bis zu 20 Gew.-%, bezogen auf das gelöste Diisocyanat betragen. Außer diesen bereits während der Phosgenierungsreaktion entstehenden Nebenprodukten entstehen durch Polymerisation und sonstige Nebenreaktionen während der destillativen Aufarbeitung der bei der Phosgenierung anfallenden Lösungen weitere Anteile an Verunreinigungen, die entfernt werden müssen.

Bei der Durchführung des erfindungsgemäßen Verfahrens werden die genannten Rohlösungen zunächst von der Hauptmenge des vorliegenden Lösungsmittels und gegebenenfalls einer Teilmenge des gebildeten Diisocyanats destillativ befreit, so daß Konzentrate entstehen, die noch einen Restgehalt an Lösungsmittel der genannten Art von 2 bis 15 Gew.-% aufweisen. Die Herstellung dieser Konzentrate erfolgt im allgemeinen bei einer Destillationstemperatur von 100 bis 180°C und unter einem Druck von 5 bis 200 mbar. Vorzugsweise wird diese Destillation kontinuierlich in den üblichen kontinuierlich betriebenen Destillationsanlagen durchgeführt.

Als nächster Schritt erfolgt bei der Durchführung des erfindungsgemäßen Verfahrens eine intensive Durchmischung der genannten Konzentrate mit bei Raumtemperatur flüssigen aliphatischen Kohlenwasserstoffen und/oder mit bei Raumtemperatur flüssigen aliphatischen Ethern als Extraktionsmittel.

Geeignete Extraktionsmittel sind insbesondere aliphatische Kohlenwasserstoffe mit mindestens 5, vorzugsweise 6 bis 12 Kohlenstoffatomen bzw. entsprechende Kohlenwasserstoffgemische oder aliphatische Ether mit mindestens 4, vorzugsweise 4 bis 12 Kohlenstoffatomen oder entsprechende Ethergemische oder Gemische von aliphatischen Kohlenwasserstoffen und Ethern der genannten Art. Geeignete Extraktionsmittel sind beispielsweise n-Hexan, i-Octan, den genannten Definitionen entsprechende Benzinfraktionen, Diethylether, die isomeren Butylether, Methyl-n-heptylether und/oder Ethyl-n-decyl-ether bzw. beliebige Gemische derartiger Flüssigkeiten. Die beispielhaft genannten aliphatischen Kohlenwasserstoffe sind gegenüber den Ethern bevorzugt.

Die beispielhaft genannten Extraktionsmittel werden, bezogen auf das Konzentrat, in einer Menge von 50 bis 3000 Gew.-%, vorzugsweise 100 bis 2000 Gew.-% eingesetzt.

Die Durchmischung des Konzentrats mit dem Extraktionsmittel erfolgt im allgemeinen innerhalb des Temperaturbereichs von -20°C bis 130°C, vorzugsweise bei 10 bis 100°C.

Im allgemeinen entsteht hierbei spontan ein zweiphasiges, aus zwei flüssigen Phasen bestehendes Gemisch, welches nach Phasenabscheidung in eine spezifisch leichtere und eine spezifisch schwerere Phase getrennt werden kann. Die Bildung eines zweiphasigen Systems kann in besonderen Fällen jedoch auch durch eine Abkühlung des Gemischs des Konzentrats mit dem Extraktionsmittel begünstigt werden. So ist es beispielsweise möglich, die Durchmischung bei ca. 80 - 130°C vorzunehmen und anschließend das Gemisch auf eine tiefere Temperatur beispielsweise im Temperaturbereich von 10 bis 50°C abzukühlen. In dem sich bildenden zweiphasigen System bildet die spezifisch leichtere Phase im allgemeinen die Hauptphase und die spezifisch schwerere Phase die Nebenphase, wobei das Volumenverhältnis weitgehend von der Menge des eingesetzten Extraktionsmittels abhängt. Die Phasentrennung kann bei der Durchführung des erfindungsgemäßen Verfahrens nach an sich bekannten Methoden, beispielsweise durch Ablassen der schwereren Phase, Dekantieren, Abhebern oder andere geeignete Methoden der Phasentrennung vorgenommen werden. Die Hauptmenge des in reiner Form zu gewinnenden Diisocyanats liegt dann in der spezifisch leichteren Hauptphase vor. Weitere Bestandteile der spezifisch leichteren Phase sind ein Teil des noch vorliegenden Lösungsmittels, welches bei der Phosgenierung eingesetzt worden war und die Hauptmenge des eingesetzten Extraktionsmittels. Die spezifisch schwerere Phase besteht in erster Linie aus einem Teil des bei Phosgenierung eingesetzten und bei der Herstellung des Konzentrats nicht entfernten Lösungsmittels, den sich während der Phosgenierung und der Herstellung des Konzentrats gebildeten Nebenprodukten und geringen Mengen an monomerem Diisocyanat. Um auch diese Restmengen an monomerem Diisocyanat in reiner Form zu gewinnen kann die spezifisch schwerere Phase einer erneuten Extraktion mit einem Extraktionsmittel der genannten Art zugeführt werden.

Gemäß einer bevorzugten Ausführungsform des erfindungsgemäßen Verfahrens erfolgt die Durchmischung des Konzentrats mit dem Extraktionsmittel und die anschließende Phasentrennung, d.h. die Extraktion des Konzentrats in kontinuierlicher Fahrweise unter Verwendung der üblichen kontinuierlich arbeitenden Gegenstromextraktionsapparaturen.

Als Ergebnis verbleiben nach der ein- bzw. mehrstufigen Extraktion eine bzw. mehrere, gegebenenfalls miteinander zu vereinigende, spezifisch leichtere Extraktphase(n) die das gereinigte Diisocyanat enthält bzw. enthalten und eine homogene zweite Phase, die als hochviskose Flüssigphase ausgetragen werden kann. Die flüssige Konsistenz der auszutragenden spezifisch schwereren Phase kann durch geeignete Wahl der Extraktionstemperatur innerhalb der obengenannten Bereiche sichergestellt werden.

Zur Gewinnung der Diisocyanate in reiner Form werden die in der bzw. den spezifisch leichteren Extraktphasen vorliegenden inerten Flüssigkeiten (Restmengen an Lösungsmittel und Extraktionsmittel) destillativ bei einer Temperatur von 50 bis 180°C und einem Druck von 20 bis 1000 mbar entfernt, so daß das gereinigte Diisocyanat als Destillationsrückstand anfällt. Auch diese destillative Entfernung der inerten Flüssigkeiten kann gewünschtenfalls in kontinuierlicher Fahrweise in an sich bekannten kontinuierlich arbeitenden Destillationsapparaturen erfolgen. Gewünschtenfalls kann das als Destillationsrückstand anfallende, gereinigte Diisocyanat einer weiteren Feindestillation unterzogen werden. Jedoch auch ohne eine Feindestillation weisen die als Destillationsrückstände anfallenden Diisocyanate im allgemeinen bereits einen Reinheitsgrad von mindestens 90 Gew.-% auf.

Es ist ein besonderer Vorteil des erfindungsgemäßen Verfahrens, daß bei der geschilderten Abtrennung der monomeren Diisocyanate aus den Konzentraten bzw. Rohlösungen eine weitere Umsetzung von monomerem Diisocyanat mit oligomeren und polymeren Nebenprodukten weitgehend unterbunden wird, so daß im Vergleich zu den bekannten Aufarbeitungsmethoden des Standes der Technik, bei welchen weit höhere Temperaturen zur Anwendung kommen die Bildung von unerwünschten Nebenprodukten zurückgedrängt werden kann. Dies hat zur Folge, daß bei der erfindungsgemäßen Aufarbeitung der Rohlösungen ein wesentlich größerer Anteil des bei der Phosgenierung gebildeten Diisocyanats erhalten bleibt und in reiner Form gewonnen werden kann.

Grundsätzlich ist es auch möglich, nach dem erfindungsgemäßen Verfahren Diisocyanat-Rohlösungen aufzuarbeiten, die den erfindungsgemäß einzusetzenden Ausgangslösungen entsprechen, jedoch nicht durch Phosgenierung der den Diisocyanaten entsprechenden Diamine sondern auf anderem Wege in Gegenwart von inerten organischen Lösungsmitteln der genannten Art erhalten worden sind. Derartige andere Methoden der Herstellung von organischen Diisocyanaten sind beispielsweise die Umsetzung der den Diisocyanaten entsprechenden Diamine mit Oxalylchlorid als Phosgenersatz oder die Herstellung von Diisocyanatotoluol durch Umsetzung von Dinitrotoluol mit Kohlenmonoxid in Gegenwart geeigneter Katalysatoren.

Die nachfolgenden Beispiele dienen der weiteren Erläuterung des erfindungsgemäßen Verfahrens. Alle Prozentangaben beziehen sich auf Gewichtsprozente.

## Beispiele

Die in den nachfolgenden Beispielen eingesetzten Rohlösungen wurden durch Phosgenierung der entsprechenden Diamine im ortho-Dichlorbenzol bzw. Monochlorbenzol und nachträgliche destillative Entfernung der Hauptmenge des eingesetzten Lösungsmittels und einer Teilmenge des gebildeten Diisocyanats hergestellt. Die ursprünglichen Lösungen der Phosgenierungsprodukte wiesen einen Gehalt von 90 % (TDI) bzw. von 96 % (HDI) an ortho-Dichlorbenzol bzw. 90 % (IPDI) an Monochlorbenzol auf. Aus den Rohlösungen wurden alle niedrigsiedenden Anteile wie Chlorwasserstoff und überschüssiges Phosgen sowie der größte Teil des Lösungsmittels und eine Teilmenge des gebildeten Diisocyanats destillativ bei einer Temperatur von 140°C und einem Druck 50 mbar entfernt, so daß die in der Tabelle angegebenen Restmengen an ortho-Dichlorbenzol bzw. Monochlorbenzol vorlagen. Zur Extraktion der so hergestellten Konzentrate wurden diese mit unterschiedlichen Extraktionsmitteln (vgl. Tabelle) bei unterschiedlichen Temperaturen (vgl. Tabelle) in einem Laborscheidetrichter gemischt. In dem Laborscheidetrichter bildeten sich jeweils zwei Phasen aus. Nach ca. 15 Minuten wurden die beiden Phasen getrennt, indem die schwerere Phase abgelassen wurde. Die so abgetrennte schwerere Phase wurde in manchen Beispielen weiteren Extraktionen unterworfen, indem sie jeweils wieder mit frischem Extraktionsmittel versetzt wurde. Auch hierbei bildeten sich erneut zwei Phasen aus, wobei erneut die schwerere Phase abgelassen und gegebenenfalls erneut als Ausgangsmaterial für eine weitere Extraktion verwendet wurde. Die Reindarstellung der extrahierten Diisocyanate erfolgte schließlich durch Abdestillieren der in den leichteren Phasen vorliegenden Lösungs- und Extraktionsmittel. Weitere Einzelheiten sind der nachfolgenden Tabelle zu entnehmen.

**Tabelle**

| Bsp | Diisocyanat | Extraktions-stufe | Extraktions-mittel | Extraktions-mittel Menge | Diisocyanatkonzentrat | | | Extraktionstemperatur | | g Diiso-cyanat extra-hiert | % Diiso-cyanat extra-hiert |
|---|---|---|---|---|---|---|---|---|---|---|---|
| | | | | | Mengemonome-res ** Diiso-cyanat % | | o-Dich-lorben-zol % | Misch-ung | Phasen-trenn-ung | | |
| 1 | Diisocyanato-toluol * | 1 | i-Octan | 150 g | 20 g | 87,6 | 7,3 | 20 | 20 | 14,9 | 85,1 |
| 2 | 1,6-Diiso cyanatohexan | 1 | i-Octan | 300 g | 20 g | 90,2 | 4,6 | 20 | -10 | 16,2 | 89,8 |
| 3 | Diisocyanato-toluol * | 1 | Petrolether Sdb 30-60°C | 20 g | 20 g | 74,6 | 14,9 | 20 | 20 | 6,3 | |
| | | 2 | " | 20 g | | | | 30 | 30 | 4,3 | |
| | | 3 | " | 20 g | | | | 30 | 30 | 1,7 | |
| | | 4 | " | 20 g | | | | | | 0,7 | 87,1 |
| 4 | Diisocyanato-toluol * | 1 | i-Octan | 20 g | 20 g | 58,5 | 2,7 | 90 | 20 | 7,2 | |
| | | 2 | " | 20 g | | | | 90 | 90 | 3,4 | |
| | | 3 | " | 20 g | | | | 90 | 90 | 0,2 | 92,3 |
| 5 | 1-Isocyanato-3,3,5-trimethyl-5-isocyanato-methyl-cyclo-hexan (IPDI) | 1 | n-Octan | 61 g | 20 g | 70 g | 7,2 | 80 | 80 | 13,1 | 93,4 |

*) 80% 2,4- und 20 % 2,6-Isomeres
**) gaschromatographisch bestimmt

**Patentansprüche**

1. Verfahren zur Gewinnung von reinen Diisocyanaten ausgewählt aus der Gruppe bestehend aus (i) 2,4-Diisocyanatotoluol und dessen Gemischen mit bis zu 35 Gew.-%, bezogen auf Gesamtgemisch, an 2,6-Diisocyanatotoluol, (ii) 1,6-Diisocyanatohexan und (iii) 1-Isocyanato-3,3,5-trimethyl-5-isocyanatomethyl-cyclohexan aus konzentrierten Lösungen der Phosgenierprodukte der den Diisocyanaten zugrundeliegenden Diamine in technischem Chlorbenzol und oder technischem ortho-Dichlorbenzol, dadurch gekennzeichnet, daß man die bei der Phosgenierung der den Diisocyanaten zugrundeliegenden Diamine in den genannten Lösungsmitteln anfallenden, einen Lösungsmittelgehalt von 40 bis 98 Gew.-% aufweisenden Lösungen der Phosgenierungsprodukte destillativ von der Hauptmenge des Lösungsmittels bis zu einem Restgehalt von 2 bis 15 Gew.-% und gegebenenfalls einer Teilmenge des gebildeten Diisocyanats befreit, die so erhaltenen Konzentrate bei Temperaturen zwischen -20°C und 130°C mit bei Raumtemperatur flüssigen aliphatischen Kohlenwasserstoffen und/oder mit bei Raumtemperatur flüssigen aliphatischen Ethern als Extraktionsmittel unter Einhaltung eines Gewichtsverhältnisses von Extraktionsmittel zu Konzentrat von 0,5 : 1 bis 30 : 1 innig unter Bildung eines flüssigen, zweiphasigen Gemischs vermischt, die sich bildenden Flüssigphasen nach Phasenabscheidung voneinander trennt, das reine Diisocyanat aus der spezifisch leichteren Flüssigphase durch destillative Entfernung des in ihr enthaltenen Lösungs- und Extraktionsmittels gewinnt und gegebenenfalls die abgetrennte, spezifisch schwere Phase einer erneuten Extraktion zuführt.

2. Verfahren gemäß Anspruch 1, dadurch gekennzeichnet, daß man die Herstellung des bei der Extraktion einzusetzenden Konzentrats durch destillative Entfernung der Hauptmenge des vorliegenden Lösungsmittels kontinuierlich in einer kontinuierlich betriebenen Destillationseinlage durchführt.

3. Verfahren gemäß Anspruch 1 und 2, dadurch gekennzeichnet, daß man die Durchmischung des Konzentrats mit dem Extraktionsmittel und die Phasentrennung kontinuierlich in einer kontinuierlich betriebenen Gegenstromextraktionsanlage durchführt.

4. Verfahren gemäß Anspruch 1 bis 3, dadurch gekennzeichnet, daß man die destillative Entfernung des Lösungs- und Extraktionsmittels aus der abgetrennten, spezifisch leichteren Flüssigphase kontinuierlich in einer kontinuierlich betriebenen Destillationsanlage durchführt.

5. Verfahren gemäß Anspruch 1 bis 4, dadurch gekennzeichnet, daß man als Extraktionsmittel aliphatische Kohlenwasserstoffe mit 6 bis 12 Kohlenstoffatomen und/oder aliphatische Ether mit 4 bis 12 Kohlenstoffatomen verwendet.

## Claims

1. A process for the recovery of pure diisocyanates selected from the group consisting of (i) 2,4-diisocyanatotoluene and mixtures thereof with up to 35 % by weight, based on the mixture as a whole, of 2,6-diisocyanatotoluene, (ii) 1,6-diisocyanatohexane and (iii) 1-isocyanato-3,3,5-trimethyl-5-isocyanatomethyl cyclohexane from concentrated solutions of the phosgenation products of the diamines on which the diisocyanates are based in technical chlorobenzene and/or technical ortho-dichlorobenzene, characterized in that the solutions of the phosgenation products which accumulate in the phosgenation of the diamines forming the basis of the diisocyanates in the solvents mentioned and which have a solvent content of 40 to 98 % by weight are freed by distillation from most of the solvent to a residual content of 2 to 15 % by weight and, optionally, a partial amount of the diisocyanate formed, the concentrates thus obtained are thoroughly mixed at temperatures of from -20°C to 130°C with aliphatic hydrocarbons liquid at room temperature and/or with aliphatic ethers liquid at room temperature as extractants, the ratio by weight of extractant to concentrate being kept at 0.5 : 1 to 30 : 1, to form a liquid two-phase mixture, the liquid phases formed are separated from one another after phase separation, the pure diisocyanate is recovered from the liquid phase of lower specific gravity by removal of the solvent or extractant present therein by distillation and the phase of higher specific gravity separated off is optionally delivered to another extraction.

2. A process as claimed in claim 1, characterized in that the preparation of the concentrate to be used in the extraction by removal of most of the solvent present by distillation is carried out continuously in a continuous distillation plant.

3. A process as claimed in claims 1 and 2, characterized in that the mixing of the concentrate with the extractant and phase separation are carried out continuously in a continuous countercurrent extraction plant.

4. A process as claimed in claims 1 to 3, characterized in that the removal of the solvent or extractant by distillation from the liquid phase of lower specific gravity separated off is carried out continuously in a continuous distillation plant.

5. A process as claimed in claims 1 to 4, characterized in that aliphatic hydrocarbons containing 6 to 12 carbon atoms and/or aliphatic ethers containing 4 to 12 carbon atoms are used as the extractant.

## Revendications

1. Procédé pour récupérer des diisocyanates purs, choisis dans l'ensemble constitué par (i) le 2,4-diisocyanatotoluène et ses mélanges avec jusqu'à 35 % en poids, sur la base du mélange total, de 2,6-diisocyanatotoluène, (ii) le 1,6-diisocyanatohexane et (iii) le 1-isocyanato-3,3,5-triméthyl-5-isocyanatométhyl-cyclohexane, à partir de solutions concentrées des produits de phosgénation des diamines qui sont à la base des diisocyanates, dans du chlorobenzène technique et/ou dans l'ortho-dichlorobenzène technique, procédé caractérisé en ce qu'on soumet les solutions des produits de phosgénation, obtenues lors de la phosgénation des diamines qui sont à la base des diisocyanates dans les solvants cités, et qui présentent une teneur moyenne en solvants de 40 à 98 % en poids, à une élimination, par distillation, de la majeure partie au solvant, jusqu'à une teneur résiduelle de 2 à 15 % en poids et éventuellement on élimine également une fraction du diisocyanate formé; on mélange de façon intime les concentrés ainsi obtenus, à des températures comprises entre -20°C et +130°C, avec des hydrocarbures aliphatiques liquides à la température ambiante et/ou avec des éthers-oxydes aliphatiques liquides à la température ambiante, comme agent d'extraction, en maintenant un rapport pondéral entre l'agent d'extraction et le concentré allant de 0,5 : 1 à 30 : 1, en formant un mélange liquide à deux phases; après la séparation spontanée des phases, on sépare l'une de l'autre les phases liquides qui se sont formées, on récupère le diisocyanate pur, à partir de la phase liquide spécifiquement plus légère, en éliminant par distillation le solvant et l'agent d'extraction contenus dans cette phase légère, et éventuellement on achemine la phase séparée, spécifiquement plus lourde, vers une nouvelle extraction.

2. Procédé selon la revendication 1, caractérisé en ce qu'on conduit en continu, dans une installation de distillation fonctionnant en continu, la préparation du concentré, à utiliser pour l'extraction, en éliminant par distillation la majeure partie du solvant présent.

3. Procédé selon les revendications 1 et 2, caractérisé en ce qu'on conduit le mélangeage du concentré avec l'agent d'extraction et la séparation des phases en opérant en continu dans une installation d'extraction à contre-courant fonctionnant en continu.

4. Procédé selon les revendications 1 à 3, caractérisé en ce qu'on conduit l'élimination, par distillation, du solvant et de l'agent d'extraction de la phase liquide, spécifiquement plus légère, séparée, en opérant en continu dans une installation de distillation fonctionnant en continu.

5. Procédé selon les revendications 1 à 4, caractérisé en ce qu'on utilise comme agent d'extraction des hydrocarbures aliphatiques comportant 6 à 12 atomes de carbone et/ou des éthers-oxydes comportant 4 à 12 atomes de carbone.